(19) Europäisches Patentamt
European Patent Office
Office européen des brevets

(11) Publication number: 0 095 092 B1

# (12) EUROPEAN PATENT SPECIFICATION

(45) Date of publication of patent specification: 30.12.86

(51) Int. Cl.$^4$: C 07 D 457/04

(21) Application number: 83104636.2

(22) Date of filing: 11.05.83

## (54) Process for the preparation of lysergic acid esters.

(30) Priority: 26.05.82 DE 3220199

(43) Date of publication of application:
30.11.83 Bulletin 83/48

(45) Publication of the grant of the patent:
30.12.86 Bulletin 86/52

(84) Designated Contracting States:
AT BE CH DE FR GB IT LI LU NL SE

(56) References cited:
CH-A- 452 538
FR-A-1 115 726
GB-A- 759 105

S.Patai: The chemistry of acid derivatives", part 1, supplement B, 1979, published by John Wiley & Sons, New York, USA.

(73) Proprietor: SCHERING AKTIENGESELLSCHAFT
Berlin und Bergkamen
Müllerstrasse 170/178 Postfach 65 03 11
D-1000 Berlin 65 (DE)

(72) Inventor: Sauer, Gerhard, Dr.
Königsbacher Zeile 41A
D-1000 Berlin 28 (DE)
Inventor: Haffer, Gregor, Dr.
Mörchinger Strasse 79
D-1000 Berlin 37 (DE)

EP 0 095 092 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

The present invention concerns a new method for preparing lysergic acid esters.

Lysergic acid esters are intermediates for the conventional production of pharmacologically active compounds and also of each other. Thus, the known lisuride hydrogen maleate (US—A—3,251,846) can be prepared, via the intermediate stage of the azide, for example according to the process described by Zikan [V. Zikan et al., Coll. Czechoslov. Chem. Commun., *25*: 1922 (1960)], using any of the esters prepared by the process of this invention.

In general, an acid amide is prepared from the ester. However, it is also possible to conduct the inverse reaction, i.e., the conversion of an acid amide into the corresponding ester, in one step, cf. S. Patai: "The Chemistry of Acid Derivatives", part I, Suppl. B (1979), pages 418—419, paragraph 6. For this purpose, the acid amide is dissolved in an alcohol and a strong acid is added thereto. In most cases, a high acid concentration is required, for example, by introducing hydrogen chloride for several hours. This reaction is ordinarily accomplished under boiling heat of the alcohol employed, in some cases even in a sealed tube (Weygand-Hilgetag, "Organisch-chemische Experimentierkunst" [Experimenting Art in Organic Chemistry] Leipzig 1970, p. 352).

The reaction of the corresponding amide or nitrile with an alcohol to yield the alkyl ester is described in IR—A—1.115.726 and GB—A—759105.

On the other hand, it is also known that lysergic acid and its derivatives, especially the esters, are easily rearranged under strongly acidic conditions into entirely useless benz[c,d]indolines [A. Stoll and T. Petrzilka, Helv. Chim. Acta *36*: 1125 (1953)]. This poses a major problem in acid catalyzed reactions preparing or using such esters.

Alkaline saponification of lysergic acid amides likewise takes place under rather drastic conditions with 4—7N potassium hydroxide solution under heating. It does not yield pure lysergic acid which when would first have to be esterified in a second step (see DE—B—2,610,859).

Summary of the Invention

It is an object of the present invention to provide a process that produces the desired lysergic acid esters in the pure stage in a high yield, e.g., typically at least about 80 molar% of theory.

It has now been found surprisingly that it is possible to convert lysergic acid and/or isolysergic acid amides, in the presence of an inorganic or organic acid, into the natural lysergic acid ester of 8β-configuration, in a smooth reaction, there additionally occurring in case of the isolysergic acid amide reactant, a surprising isomerization to the lysergic acid ester. The yields are practically quantitative.

Upon further study of the specification and appended claims, further objects and advantages of this invention will become apparent to those skilled in the art.

These objects have been achieved by providing a process for the preparation of lysergic acid esters of the formula

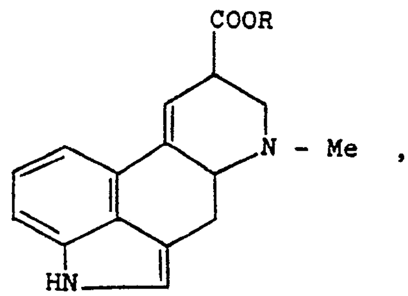

wherein R is alkyl of up to 3 carbon atoms, comprising treating lysergic acid and/or isolysergic acid amides of the formula

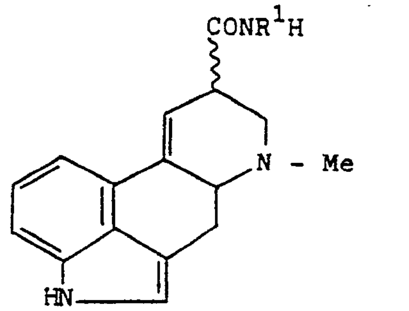

wherein $R^1$ is hydrogen or lower alkyl of up to 4 carbon atoms, optionally substituted by an OH-group, or a reaction compatible salt thereof with an acid, with an alcohol of the formula ROH wherein R is alkyl of up to 3 carbon atoms, at temperatures of 0° to 65°C for a period of 2 to 30 hours in the presence of an acid at a pH value of 0—1.

Detailed Discussion

In order to conduct the process, a lysergic acid amide or isolysergic acid amide or a mixture of both is dissolved in the corresponding alcohol, e.g., methanol, ethanol, n-propanol, or isopropanol. Generally, amounts of the amide relative to the alcohol are 1—10 parts by weight, i.e., usually an excess of alcohol is utilized. Suitable acids include for example sulfuric acid, hydrochloric acid, perchloric acid, p-toluenesulfonic acid, trifluoroacetic acid, trifluoromethanesulfonic acid, etc., or an acid in the form of an acidic ion exchange resin, e.g., sulfonic acids like $RSO_3H$, wherein R is the resin residue.

The acid is added to the alcohol solution in a quantity so that the pH value of the reaction solution is about 0—1. The reaction is carried out at 0° to the boiling temperature of the reaction mixture, generally up to 65°C; suitably, the temperature range is 20—55°C. The reaction temperature is maintained for a relatively long period of time. Depending on the acid concentration and reaction temperature the reaction period is 2 hours, usually at most 30 hours and, in the normal case, is completed after about 16 hours. Preferably, the reaction is conducted under anhydrous conditions.

Subsequently the reaction mixture is worked up by using known methods, such as washing, extraction, precipitation, crystallization, etc.

Suitable as starting materials in the reaction of this invention are all free and N-substituted lysergic acid amides and isolysergic acid amides of the formula

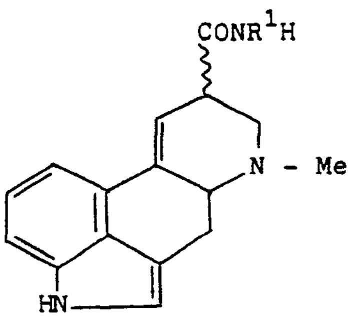

,

wherein $R^1$ is hydrogen or lower alkyl of up to 4 carbon atoms, which can optionally be substituted by an OH-group. Examples in this connection include lysergic acid amide, isolysergic acid amide, ergometrine, ergometrinine, etc. All of these starting materials are known or readily conventionally preparable.

Lower alkyl includes, for example, methyl, ethyl, n-propyl, isopropyl, butyl, etc., but especially methyl.

Acid salts of the amides can equivalently be used as starting materials, e.g., the hydrochloride, tartrate, hydrogen maleate, methane sulphonate and hydrogen phosphate.

Without further elaboration, it is believed that one skilled in the art can, using the preceding description, utilize the present invention to its fullest extent. The following preferred specific embodiments are, therefore, to be construed as illustrative. In the following examples, all temperatures are set forth uncorrected in degrees Celsius; unless otherwise indicated, all parts and percentages are by weight.

Example 1

A suspension is prepared from 5.0 g of isolysergic acid amide in 80 ml of anhydrous methanol; the mixture is cooled to about −50°C and, under agitation, 40 ml of a 13N solution of hydrogen chloride in anhydrous methanol is added thereto. The clear solution is allowed to warm up to room temperature and further stirred overnight. The primary amount of the thus-formed ester hydrochloride is thus crystallized. By cooling in an ice bath and adding ethyl acetate, the crystallization is completed, and the precipitate is vacuum-filtered.

Yield: 5.2 g (84% of theory) of lysergic acid methyl ester, hydrochloride.

$[\alpha]_D = +94°$ (0.5% in methanol).

Example 2

Starting with 5.0 g of lysergic acid amide, lysergic acid methyl ester is obtained as the hydrochloride in an 86% yield according to Example 1.

$[\alpha]_D = +94°$ (0.5% in methanol).

Example 3

A mixture of 2.5 g of lysergic acid amide and 2.5 g of isolysergic acid amide in 500 ml of ethanol is heated with 100 g of p-toluenesulfonic acid for 4 hours to 50°C. Then half of the solvent is removed by distillation, the residue is poured into a mixture of 200 ml of concentrated ammonia and ice, and extracted with methylene chloride. The organic phase is dried with sodium sulfate and evaporated, thus obtaining after crystallization from ethanol 4.45 g (81% of theory) of lysergic acid methyl ester.

$[\alpha]_D = +68°$ (0.5% in chloroform).

Example 4

A solution is prepared of 4.4 g of ergometrine maleate (10 millimoles) in 300 ml of methanol under nitrogen; 10 g of p-toluenesulfonic acid is added thereto, and the mixture is heated in a sealed flask overnight to 50°C. The solution is concentrated under vacuum, taken up in methylene chloride, and combined with concentrated ammonia solution until an alkaline reaction is obtained. After separating the phases and further extraction of the aqueous phase, the organic phases are dried with sodium sulfate and evaporated. The residue is crystallized from methylene chloride and diisopropyl ether.

Yield: 2.5 g (89% of theory) of lysergic acid methyl ester.

$[\alpha]_D = +84°$ (0.5% in chloroform).

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

**Claims**

1. A process for preparing a lysergic acid ester of the formula

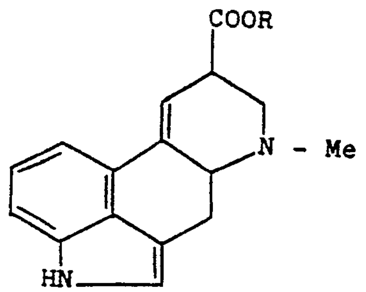

,

wherein R is alkyl of up to 3 carbon atoms, comprising reacting a lysergic acid or isolysergic acid amide of the formula

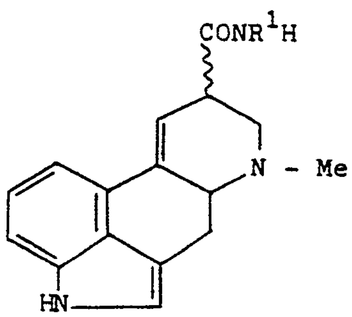

,

wherein $R^1$ is hydrogen or lower alkyl of up to 4 carbon atoms optionally substituted by an OH-group, or a reaction compatible salt thereof with an acid, with an alcohol of the formula ROH wherein R is alkyl of up to 3 carbon atoms, at a temperature of 0° to 65°C for a period of 2 to 30 hours in the presence of an acid or an acidic ion exchange resin at a pH value of about 0—1.

2. A process of claim 1 wherein the starting material amide is a lysergic acid amide.

3. A process of claim 1 wherein the starting material amide is an isolysergic acid amide.

4. A process of claim 1 wherein the starting material amide is a mixture of lysergic and isolysergic acid amides.

5. A process of claim 1 wherein $R^1$ is methyl.

6. A process of claim 1 wherein the pH is provided by adding an acid.

7. A process of claim 1 wherein the pH is effectively provided by using an acidic ion exchange resin.

8. A process of claim 1 wherein the acid is HCl or p-toluenesulfonic acid.

9. A process of claim 1 wherein the reaction temperature is 20—55°C.

10. A method of claim 1 wherein the reaction time is 16—2 hours.

**Patentansprüche**

1. Verfahren zur Herstellung eines Lysergsäureesters der Formel

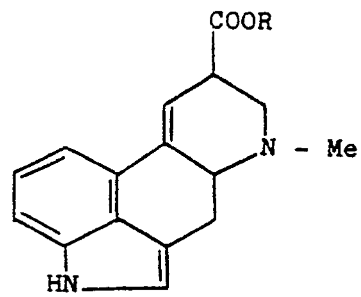

,

worin R Alkyl mit bis zu 3 Kohlenstoffatomen bedeutet, umfassend die Umsetzung eines Lysergsäure- oder Isolysergsäureamids der Formel

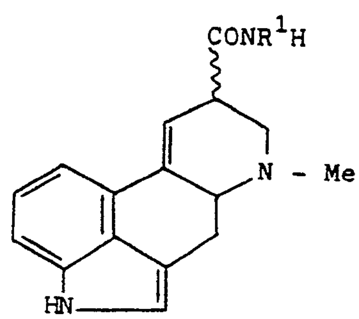

,

worin $R^1$ Wasserstoff oder Niedrigalkyl mit bis zu 4 Kohlenstoffatomen, das gegebenenfalls durch eine OH-Gruppe substituiert ist, bedeutet, oder eines reaktionsverträglichen Salzes davon mit einer Säure, mit einem Alkohol der Formel ROH, worin R Alkyl mit bis zu 3 Kohlenstoffatomen bedeutet, bei einer Temperatur von 0° bis 65°C während eines Zeitraumes von 2 bis 30 Stunden in Gegenwart einer Säure oder eines sauren Ionenaustauscherharzes bei einem pH-Wert von etwa 0—1.

2. Verfahren nach Anspruch 1, wobei das Ausgangsmaterial-Amid ein Lysergsäureamid ist.

3. Verfahren nach Anspruch 1, wobei das Ausgangsmaterial-Amid ein Isolysergsäureamid ist.

4. Verfahren nach Anspruch 1, wobei das Ausgangsmaterial-Amid ein Gemisch von Lysergsäure- und Isolysergsäureamiden ist.

5. Verfahren nach Anspruch 1, wobei $R^1$ Methyl ist.

6. Verfahren nach Anspruch 1, wobei der pH durch Zugabe einer Säure eingestellt wird.

7. Verfahren nach Anspruch 1, wobei der pH durch Verwendung eines sauren Ionenaustauscherharzes wirksam eingestellt wird.

8. Verfahren nach Anspruch 1, wobei die Säure HCl oder p-Toluolsulfonsäure ist.

9. Verfahren nach Anspruch 1, wobei die Reaktionstemperatur 20—55°C beträgt.

10. Verfahren nach Anspruch 1, wobei die Reaktionszeit 16 bis 2 Stunden beträgt.

**Revendications**

1. Procédé de préparation d'un ester de l'acide lysergique répondant à la formule:

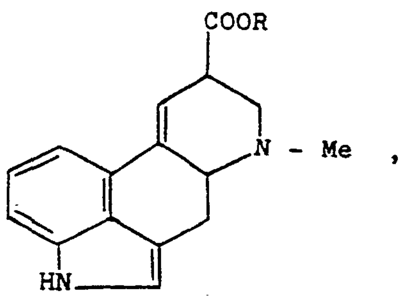

dans laquelle R représente un radical alkyle contenant au plus 3 atomes de carbone, procédé selon lequel on fait réagir un amide de l'acide lysergique ou de l'acide isolysergique répondant à la formule:

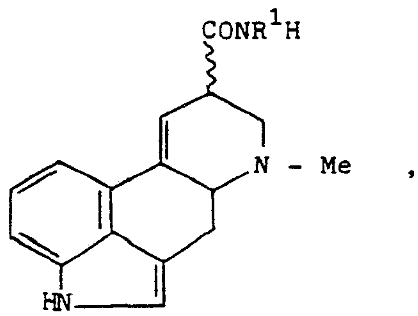

dans laquelle $R^1$ représente l'hydrogène ou un radical alkyle contenant au plus 4 atomes de carbone et portant éventuellement un radical —OH, ou un sel, compatible avec la réaction, dérivant d'un tel composé et d'un acide, avec un alcool de formule ROH dans lequel R représente un radical alkyle contenant au plus 3 atomes de carbone, à une température de 0 à 65°C, pendant un temps de 2 à 30 heures, en présence d'un acide ou d'une résine échangeuse d'ions acide, à un pH d'environ 0 à 1.

2. Procédé selon la revendication 1 caractérisé en ce que l'amide dont on part est le lysergamide.

3. Procédé selon la revendication 1 caractérisé en ce que l'amide dont on part est l'isolysergamide.

4. Procédé selon la revendication 1 caractérisé en ce que l'amide dont on part est un mélange de lysergamide et d'isolysergamide.

5. Procédé selon la revendication 1 caractérisé en ce que $R^1$ représente le radical méthyle.

6. Procédé selon la revendication 1 caractérisé en ce que le pH est atteint par addition d'un acide.

7. Procédé selon la revendication 1 caractérisé en ce qu'on règle efficacement le pH en utilisant une résine échangeuse d'ions acide.

8. Procédé selon la revendication 1 caractérisé en ce que l'acide est HCl ou l'acide p-toluènesulfonique.

9. Procédé selon la revendication 1 caractérisé en ce que la température réactionnelle est comprise entre 20 et 55°C.

10. Procédé selon la revendication 1 caractérisé en ce que la durée de la réaction est comprise entre 16 et 2 heures.